# EUROPEAN PATENT SPECIFICATION

(11) **EP 2 077 812 B1**
(45) Date of publication and mention of the grant of the patent: **26.03.2014**
(21) Application number: 07824291.4
(22) Date of filing: 24.10.2007
(51) Int. Cl.: A61F 13/08, A61F 13/06

(54) **MULTI DIRECTIONAL STRETCH TUBULAR BANDAGES**
MULTIDIREKTIONALE DEHNBARE RÖHRENFÖRMIGE BANDAGEN
PANSEMENTS TUBULAIRES EXTENSIBLES DANS PLUSIEURS DIRECTIONS

(30) Priority: 03.11.2006 GB 0621926
(43) Date of publication of application: 15.07.2009
(73) Proprietor: Medlock Medical Limited, Oldham OL1 3HS (GB)
(72) Inventor: LEEMING, Raymond Charles, Oldham OL2 7AR (GB); FERNANDEZ, Damian Antonio, Oldham OL4 3NX (GB)
(74) Representative: Wilson Gunn
(86) International application number: PCT/GB2007/004043
(87) International publication number: WO 2008/053159

(56) References cited:
- EP-A- 1 561 846
- WO-A-01/80798
- DE-A1- 3 028 381
- FR-A- 2 432 867
- FR-A- 2 588 890
- FR-A- 2 633 512
- FR-A- 2 801 495

## Description

This invention relates to improvements in stretchable knitted tubular bandages for use in the applications support, retention, undercast and compression. These bandages are well known and are used in the treatment of a variety of conditions, usually affecting the arms or legs of a patient, such as injuries, disease or other uses requiring a gentle but firm inward pressure. Retention bandages may be specially for securing primary dressings on wounds or ulcers. It could be used as lightweight bandage for the treatment of atopic eczema using the Wet Wrapping, Dry Wrapping or Patch Wrapping treatment therapy. Stretchable knit tubular bandages may be used as an undercast stockinette.

Before 1960, crepe bandages were widely used as the creped fabric has some elasticity, and if the bandage is applied in a stretched state, is effective in providing some inward pressure. In GB840523 is described a tubular bandage which exerts an inward pressure at all points and conforms to the shape of the part of the torso or limbs to which it is applied, comprising a parallel seamless bandage made in a continuous length and knitted so as to possess a degree of circumferential longitudinal stretch and which includes one or more circumferentially arranged evenly pitched elastic or resilient elements such as rubber filament. The elastic or resilient element is inert or limp in the non-tensioned stated of the bandage, but becomes tensioned as the bandage is extended, and when applied about a body part effects tension such as to make the bandage conform closely to the part and exert a minimal inward pressure for retention with a higher level for support at all points.

GBA2167459 describes a variant of the tubular bandage described in the above document wherein the spacing or pitch of the elastic element is variable by varying the knit density of the stocking knit so that the elastic yarns can move more closely together in the lower density regions. This enables a greater density of elastic yarn to be provided in predetermined parts of the bandage to enable increased pressure to be applied where required without folding over the bandage to provide two or more layers.

These bandage constructions have been used successfully and are sold under the Trade Marks TUBIFAST and TUBIGRIP by Medlock Medical Limited, successors to the original patentees Seton Products Ltd. However whilst a good degree of elasticity, and grip is provided laterally of the bandage, which is provided by elastic thread inlays, the elasticity in the longitudinal direction is limited to the degree of elasticity inherent in the yarns, which is considerably less than that available circumferentially from the knit loop structure. Other bandages are described in FR 2 432 867, FR 2 801 495, FR 2 588 890, FR 2 633 512, DE 3 028 381, EP 1 561 846, WO 0 180 798.

For use on straight parts of limbs, the lateral stretch is often adequate to retain dressings or topical preparations in place. However, at joints such as elbows or knees, movement of the joint caused by flexing of the limb may allow the bandage and or primary dressing applied to the limb at or close to the joint to become creased or displaced.

It is an object of the invention to provide a bandage which is capable of being extended under elastic tension in both longitudinal and lateral directions.

A stretchable bandage according to claim 1 and the invention consist of a knitted fabric comprising a plurality of yarns of a first substantially inelastic material, which are knitted together so that loops of said yarns are interconnected, characterised in that yarns of a second elastically extensible resilient material are interlooped with the yarns of said inelastic material.

The yarns of resilient material are preferably interlooped with the yarns of inelastic material in such a way that the yarns of resilient material interlooped with each of respective pairs of inelastic yarns in the respective inter knitting of the inelastic yarns. Elastic yarns may be so introduced at each second or third pair of interknitting inelastic yarns.

In a preferred embodiment, the elastic yarns may comprise elastane yarns, and the relatively inelastic yarns may comprise viscose yarns. Of course, yarns of any other suitable material may be used, including natural fibres such as cotton, manmade fibres such as nylon and other elastic materials may be used for the resilient elastic yarns that could be latex or non latex based. Both inelastic and elastic yarns may contain antimicrobial properties such as metals, alginates, polysaccharides, chitosan and/or other chemical treatments.

The bandage according to the invention is a tubular bandage, manufactured on a circular knitting machine. The latter may be modified to provide extra yarns feeds to introduce the resilient yarns to the knitting machine. It is not excluded that the bandage according to the invention comprises a flat bandage, to form an elongate strip for use in application as a wrap around or helical bandage. Nor may it exclude a bandage knit on a flat knit machine to provide a flat or tubular bandage. The bandage may be finished with an antimicrobial treatment.

The bandage of the invention has been found to be capable of resilient stretching and recovery in both the longitudinal and lateral directions, in contrast to the known comparable treatment bandages which are only stretchable to any significant extent in the lateral direction.

In a modified of the invention, the elastic yarns may be used in a conventional stockinette, plain knit or double jersey knit in place of a non-elastic yarn, say every third or fourth yarn, the other yarns being non-elastic such as viscose.

A preferred embodiment of the invention will now be described with reference to the accompanying drawings wherein:
- Figure 1: is a diagrammatic representation, on an enlarged scale of the knit structure of a bandage according to the invention; and
- Figure 2: is a fabric notation tabulation showing the knit structure of the knitted fabric of Figure 1.

In the representation of the example of a knitted fabric in a bandage according to the present invention, there is shown the loop patterns and yarn paths of a stockinette knitted fabric, knitted as a tube in a six feed circular knitting machine. In the drawing, inelastic viscose yarns are shown white, whilst elastic, stretchable and relaxable elastane yarns are shown in black, to highlight the latter.

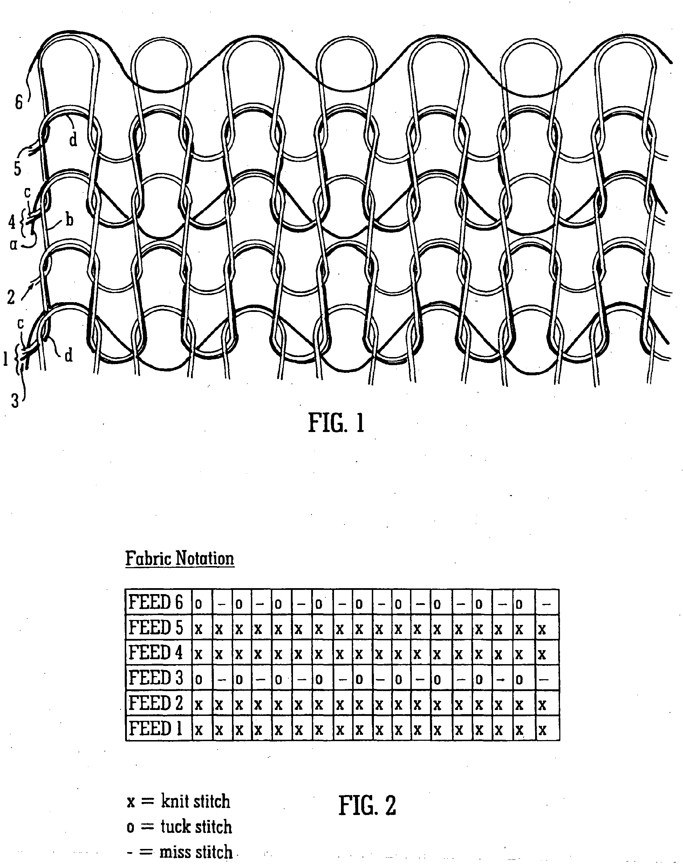

The diagram of Figure 1 shows 6 yarn feeds, numbered 1 to 6 from bottom to top. Feeds 1 and 4 are arranged to each feed a pair of yarns, namely a viscose yarn c, and an elastane yarn d. Feeds 2 and 5 each feed a single viscose yarn b, and feeds 3 and 6 each feed a single elastane yarn. The yarns c and d (pair of elastane and viscose yarn) follow the same knit path and are fully incorporated into the knit structure, the single viscose yarns b also follow a conventional yarn path in the knit, but the single elastane yarns a are effectively looped in tuck stitches about alternate stitch rows. The yarns a provide elasticity in the circumferential direction, whilst the elastane yarns d fully incorporated into the knit, provide substantially enhanced elasticity in the longitudinal direction.

From the stitch notation table in Figure 2 it will be seen that the viscose yarns from feeds 1 and 2, and feeds 4 and 5 are formed into conventional knitted stitches. It should be noted that an elastane is knitted with the viscose on every second knitted course. This ratio may be altered to suit the required end use of the bandage. To say, that the knitted elastane yarn could be introduced into the knit to be interlooped with the non-elastic yarn, be it viscose or other suitable yarn on every third, fourth or fifth knitted course.

This arrangement enables the knitted fabric to have longitudinal elasticity, as well as the lateral elastically which was provided in the prior art tubular bandages. In the latter, elastic yarns, originally rubber, more recently elastane, were simply extended along the stitch rows of the knitted fabric, with alternate stitches over and under the elastic yarns.

This enabled only stretching along the elastic yarn; the only elasticity lengthwise was due to the limited inherent stretch of the knitted fabric itself and no more than for unelasticated knits.

The knitted fabric as described is preferably knitted in tubular form, and is made up into tubular bandages. These may be used as retention bandages to secure primary dressings, or support bandages, to apply pressure to limb or other parts, and for use with emollients or other topical applications for treatment of skin conditions such as eczema or psoriasis.

The tubular bandage thus provided, is of particular value for retaining primary dressings on or close to joints such as elbows or knees, where flexure of the joint and or muscle movement has tended to displace or crease or otherwise compromise a dressing, since the longitudinal elasticity now provided for enables the movement to the accommodated, which was not possible when only radial elasticity was provided. Folds in bandages can create pressure points and this invention will reduce the incidence of it. This also enables an improvement in patient comfort and further aids patient compliance.

The fabric may also be used in strip bandages for application by winding onto a site of use. This may be formed by cutting from a tubular knitted fabric, or knitted on a flat bed. This is feasible because being tucked into the knit, anchoring of the elastane yarns is not the problem it would be with helical inlaid elastic yarns.

It will be seen that in addition to a knitted fabric, the invention also provides a tubular bandage made from the fabric, a flat strip bandage made from the fabric, and any other form of topical dressing or treatment system which can be made using the fabric according to the invention.

## Claims

1. A stretchable tubular bandage consisting of a knitted fabric comprising a plurality of yarns 'b' and 'c' of a first substantially inelastic material, which are knitted together so that the loops of said yarns 'b' and 'c' are interlooped with each other, and a plurality of yarns 'a' and 'd' of a second elastically extensible resilient material, wherein the yarns 'd' are interlooped with the yarns 'b' of said inelastic material whereby the bandage is stretchable both radially and longitudinally, and whereby the fabric obeys the following fabric notation:
| | | | | | | | | | | | | | | | | | | | |
|---|---|---|---|---|---|---|---|---|---|---|---|---|---|---|---|---|---|---|---|
| FEED 6 | Yarn 'a' | 0 | - | 0 | - | 0 | - | 0 | - | 0 | - | 0 | - | 0 | - | 0 | - | 0 | - |
| FEED 5 | Yarn 'b' | x | x | x | x | x | x | x | x | x | x | x | x | x | x | x | x | x | x |
| FEED 4 | Yarns 'c'+'d' | x | x | x | x | x | x | x | x | x | x | x | x | x | x | x | x | x | x |
| FEED 3 | Yarn 'a' | 0 | - | 0 | - | 0 | - | 0 | - | 0 | - | 0 | - | 0 | - | 0 | - | 0 | - |
| FEED 2 | Yarn 'b' | x | x | x | x | x | x | x | x | x | x | x | x | x | x | x | x | x | x |
| FEED 1 | Yarns 'c'+'d' | x | x | x | x | x | x | x | x | x | x | x | x | x | x | x | x | x | x |
| | | | | | | | | | | | | | | | | | | | |
|---|---|---|---|---|---|---|---|---|---|---|---|---|---|---|---|---|---|---|---|
| x = knit stitch 0 = tuck stitch - = miss stitch | | | | | | | | | | | | | | | | | | | |

2. A bandage according to claim 1 wherein the elastic yarns comprise elastane yarns, and the relatively inelastic yarns comprises viscose yarns.

3. A bandage according to any preceding claim wherein the elastic and the inelastic yarns contain anti-microbial additives.

## Patentansprüche

1. Dehnbarer Schlauchverband, bestehend aus einem gestrickten Gewebe, welches eine Vielzahl von Garnen 'b' und 'c' aus einem ersten im Wesentlichen unelastischen Material aufweist, die so zusammengestrickt sind, dass die Maschen der Garne 'b' und 'c' miteinander verknüpft sind, sowie eine Vielzahl von Garnen 'a' und 'd' aus einem zweiten elastisch streckbaren nachgiebigen Material aufweist, wobei die Garne 'd' mit den Garnen 'b' des unelastischen Materials verknüpft sind, wodurch der Verband sowohl in Radial- als auch Längsrichtung dehnbar ist und wodurch das Gewebe der folgenden Gewebenotation folgt:
| | | | | | | | | | | | | | | | | | | | |
|---|---|---|---|---|---|---|---|---|---|---|---|---|---|---|---|---|---|---|---|
| ZUFÜHRUNG 6 | Garn 'a' | 0 | - | 0 | - | 0 | - | 0 | - | 0 | - | 0 | - | 0 | - | 0 | - | 0 | - |
| ZUFÜHRUNG 5 | Garn 'b' | x | x | x | x | x | x | x | x | x | x | x | x | x | x | x | x | x | x |
| ZUFÜHRUNG 4 | Garn 'c'+'d' | x | x | x | x | x | x | x | x | x | x | x | x | x | x | x | x | x | x |
| ZUFÜHRUNG 3 | Garn 'a' | 0 | - | 0 | - | 0 | - | 0 | - | 0 | - | 0 | - | 0 | - | 0 | - | 0 | - |
| ZUFÜHRUNG 2 | Garn 'b' | x | x | x | x | x | x | x | x | x | x | x | x | x | x | x | x | x | x |
| ZUFÜHRUNG 1 | Garn 'c'+'d' | x | x | x | x | x | x | x | x | x | x | x | x | x | x | x | x | x | x |
| | | | | | | | | | | | | | | | | | | | |
|---|---|---|---|---|---|---|---|---|---|---|---|---|---|---|---|---|---|---|---|
| x = gestrickte Masche 0 = Fangmasche - = fallengelassene Masche | | | | | | | | | | | | | | | | | | | |

2. Verband nach Anspruch 1, wobei die elastischen Garne Elastangarne aufweisen und die relativ unelastischen Garne Viskosegarne aufweisen.

3. Verband nach einem der vorhergehenden Ansprüche, wobei die elastischen und die unelastischen Garne antimikrobielle Zusatzstoffe enthalten.

## Revendications

1. Pansement tubulaire extensible se composant d'un tissu tricoté comprenant une pluralité de fils "b" et "c" d'un premier matériau sensiblement non élastique, qui sont tricotés ensemble de sorte que les boucles desdits fils "b" et "c" sont entrelacées entre elles et une pluralité de fils "a" et "d" d'un second matériau résilient élastiquement extensible, dans lequel les fils "d" sont entrelacés avec les fils "b" dudit matériau non élastique, moyennant quoi le pansement est extensible à la fois radialement et longitudinalement, et moyennant quoi le tissu obéit à la notation de tissu suivante:
| | | | | | | | | | | | | | | | | | | | |
|---|---|---|---|---|---|---|---|---|---|---|---|---|---|---|---|---|---|---|---|
| Alimentation 6 | Fil "a" | 0 | - | 0 | - | 0 | - | 0 | - | 0 | - | 0 | - | 0 | - | 0 | - | 0 | - |
| Alimentaion 5 | Fil "b" | x | x | x | x | x | x | x | x | x | x | x | x | x | x | x | x | x | x |
| Alimentation 4 | Fils + "d" | x | x | x | x | x | x | x | x | x | x | x | x | x | x | x | x | x | x |
| Alimentation 3 | Fil "a" | 0 | - | 0 | - | 0 | - | 0 | - | 0 | - | 0 | - | 0 | - | 0 | - | 0 | - |
| Alimentation 2 | Fil "b" | x | x | x | x | x | x | x | x | x | x | x | x | x | x | x | x | x | x |
| Alimentaton 1 | Fils. + "d" | x | x | x | x | x | x | x | x | x | x | x | x | x | x | x | x | x | x |
| | | | | | | | | | | | | | | | | | | | |
|---|---|---|---|---|---|---|---|---|---|---|---|---|---|---|---|---|---|---|---|
| X = jersey 0 = maille double - = maille perdue | | | | | | | | | | | | | | | | | | | |

2. Pansement selon la revendₗcatₗon 1, dans lequel les fils élastiques comprennent des fils en élasthanne, et les fils relativement non élastiques comprennent des fils en viscose.

3. Pansement selon l'une quelconque des revendications précédentes, dans lequel les fils élastiques et non élastiques contiennent des additifs antimicrobiens.
